(19) Europäisches Patentamt
European Patent Office
Office européen des brevets

(11)  EP 3 006 085 B1

(12)  EUROPEAN PATENT SPECIFICATION

(45) Date of publication and mention
of the grant of the patent:
**14.02.2018  Bulletin 2018/07**

(51) Int Cl.:
***A61N 5/10*** *(2006.01)*      ***A61B 6/00*** *(2006.01)*

(21) Application number: **14805006.5**

(86) International application number:
**PCT/JP2014/063177**

(22) Date of filing: **19.05.2014**

(87) International publication number:
**WO 2014/192571 (04.12.2014 Gazette 2014/49)**

(54) **RADIOGRAPH ANALYSIS DEVICE, RADIATION TREATMENT SYSTEM, MARKER AREA DETECTION METHOD AND PROGRAM**

RÖNTGENSTRAHLANALYSEVORRICHTUNG, STRAHLUNGSBEHANDLUNGSSYSTEM SOWIE MARKERFLÄCHENERKENNUNGSVERFAHREN UND -PROGRAMM

DISPOSITIF D'ANALYSE RADIOGRAPHIQUE, SYSTÈME DE TRAITEMENT PAR RAYONNEMENT, PROCÉDÉ DE DÉTECTION DE ZONE DE MARQUEUR ET PROGRAMME ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **28.05.2013  JP 2013112221**

(43) Date of publication of application:
**13.04.2016  Bulletin 2016/15**

(73) Proprietors:
• **Hitachi, Ltd.**
**Tokyo 100-8280 (JP)**
• **Kyoto University**
**Kyoto-shi, Kyoto 606-8501 (JP)**

(72) Inventors:
• **TAKAHASHI, Kunio**
**Tokyo 108-8215 (JP)**

• **YAMADA, Masahiro**
**Kyoto-shi**
**Kyoto 606-8501 (JP)**
• **SAWADA, Akira**
**Nantan-shi**
**Kyoto 622-0041 (JP)**

(74) Representative: **Strehl Schübel-Hopf & Partner**
**Maximilianstrasse 54**
**80538 München (DE)**

(56) References cited:
JP-A- H0 882 880     JP-A- 2005 021 345
JP-A- 2007 505 690     JP-A- 2010 131 371
JP-A- 2011 104 149     JP-A- 2012 170 767
JP-A- 2012 170 767     US-A1- 2005 059 887
US-A1- 2006 104 420     US-A1- 2009 086 930
US-A1- 2010 104 165

**Description**

TECHNICAL FIELD

The present invention relates to a radiograph analysis device, a radiation treatment system, a marker area detection method and a program.

BACKGROUND ART

A technology of identifying a position of an affected area by previously embedding a marker having different radiation transmissivity from a human body, for example, a metal or the like, in the vicinity of the affected area of the human body, and identifying the position of the marker from a radiograph imaged by emitting radiation to the human body, is known.

For example, JP 3053389 A discloses a method of obtaining tumor marker coordinates by performing template matching through a light and shade normalization cross-correlation method in which a template image of a previously registered tumor marker is applied to image information.

[Prior Art Documents]

[Patent Document]

US 2005/059887 A1 discloses a radiograph analysis device that detects a marker area from a radiograph obtained by imaging a specimen in which a marker is embedded. The radiograph analysis device comprises a brightness relation information acquisition unit configured to acquire brightness relation information, which is generated based on information related to a quantity of radiation and which showes a relation between a brightness of the marker area and a brightness of a reference portion assumed to be a portion other than the marker. The radiograph analysis device further comprises a reference brightness acquisition unit for acquiring the brightness of the reference portion and a marker area detection unit for detecting the marker area based on the brightness relation information

JP 2012170767 A discloses a radiograph analysis device that detects a marker area from a radiograph obtained by imaging a specimen in which a marker is embedded based on the brightness relation information acquired by the brightness relation information acquisition unit and the brightness of the reference portion acquired by the reference brightness acquisition unit.

SUMMARY OF THE INVENTION

Problem to be Solved by the Invention

However, like the method disclosed in JP 3053389 A, in the method of detecting the marker using the light and shade normalization cross-correlation method, a difference between an image brightness of a marker candidate portion and an image brightness of the other portion may not be utilized effectively. That is, in the method using the light and shade normalization cross-correlation method, the marker candidate portion is determined to be a marker when the shape thereof is similar to the shape of a marker regardless of a difference between the image brightness of the marker candidate portion and the image brightness of the other portion. As the difference between the image brightness of the marker candidate portion and the image brightness of the other portion is utilized more, the probability of erroneously determining a place without a marker as being a place with a marker is increased.

The present invention provides a radiograph analysis device, a radiation treatment system, a marker area detection method and a program that are capable of more precisely utilizing a difference between an image brightness of a marker candidate portion and an image brightness of another portion.

Means for Solving the Problem

The above problems are solved by a radiograph analysis device comprising the features listed in claim 1. Advantageous embodiments are described in the dependent claims.

The brightness relation information acquisition unit may acquire a function set as the brightness relation information based on a tube voltage, a tube current and an exposure time of a radiation source and showing a determination threshold of the brightness of the marker area based on the brightness of the reference portion, and the marker area detection unit may detect a portion of the brightness equal to or smaller than the determination threshold as the marker area based on the determination threshold obtained by substituting the brightness of the reference portion acquired by the reference brightness acquisition unit for the function acquired by the brightness relation information acquisition unit.

The radiograph may be one of the radiographs obtained by simultaneously imaging the specimen in multiple directions, the brightness relation information acquisition unit may acquire the brightness relation information including a coefficient showing an influence of radiation mixed from the imaging in the other direction, the marker area detection unit may include a coefficient value setting unit configured to set a value of the coefficient; a marker area candidate extraction unit configured to extract candidates for the marker areas based on the brightness relation information having a coefficient value set by the coefficient value setting unit and the brightness of the reference portion acquired by the reference brightness acquisition unit; and a termination determination unit configured to determine whether to terminate the process of detecting the marker area by comparing the preset number of markers and the number of candidates for the marker areas extracted from the marker area candidate extraction unit, the coefficient value setting unit may vary a value of the coefficient when it is determined that the termination determination unit will not terminate the process of detecting the marker area, and the marker area candidate extraction unit may extract the candidates for the marker areas based on the value of the coefficient varied by the coefficient value setting unit.

The marker area candidate extraction unit may set a range in which a candidate for a marker area in a second image serving as a radiograph imaged in another direction is present based on the position of the candidate for the marker area in a first image serving as one of the radiographs simultaneously imaged in the multiple directions, and eliminate the candidate for the marker area in the first image from the candidates when there is no candidate for the marker area in the set range.

The marker area detection unit may include a candidate pixel determination unit configured to determine whether each pixel of the radiograph is a candidate for a pixel of the marker area based on the brightness relation information acquired by the brightness relation information acquisition unit and the brightness of the reference portion acquired by the reference brightness acquisition unit, and a template application unit configured to extract the candidate for the marker area by applying a template including a region of a marker and a region other than the marker to a determination result of the candidate pixel determination unit.

According to a second aspect of the present invention, a radiation treatment system includes any one of the above-mentioned radiograph analysis devices.

According to a third aspect of the present invention, a marker area detection method is a marker area detection method of a radiograph analysis device that detects a marker area from a radiograph obtained by imaging a specimen in which a marker is embedded, the marker area detection method including: a brightness relation information acquisition step of acquiring brightness relation information generated based on information related to the quantity of radiation and showing a relation between the brightness of the marker area and the brightness of a reference portion assumed to be a portion other than the marker; a reference brightness acquisition step of acquiring the brightness of the reference portion; and a marker area detection step of detecting the marker area based on the brightness relation information acquired in the brightness relation information acquisition step and the brightness of the reference portion acquired in the reference brightness acquisition step.

According to a fourth aspect of the present invention, a program is configured to execute the following steps in a computer serving as a radiograph analysis device configured to detect a marker area from a radiograph obtained by imaging a specimen in which a marker is embedded: a brightness relation information acquisition step of acquiring brightness relation information generated based on information related to a quantity of radiation and showing a relation between a brightness of the marker area and a brightness of a reference portion assumed to be a portion other than the marker; a reference brightness acquisition step of acquiring the brightness of the reference portion; and a marker area detection step of detecting the marker area based on the brightness relation information acquired in the brightness relation information acquisition step and the brightness of the reference portion acquired in the reference brightness acquisition step.

Effect of the Invention

According to the above-mentioned radiograph analysis device, the radiation treatment system, the marker area detection method and the program, the difference between the image brightness of the marker candidate portion and the image brightness of the other portion can be more precisely reflected.

BRIEF DESCRIPTION OF THE DRAWINGS

Fig. 1 is a schematic block diagram showing a functional configuration of a radiation treatment system according to an embodiment of the present invention.

Fig. 2 is a schematic configuration view showing a device configuration of a radiation treatment device according to the embodiment.

Fig. 3 is a schematic block diagram showing a functional configuration of a radiograph analysis device according to the embodiment.

Fig. 4 is a view for schematically showing environments of an experiment.

Fig. 5 is a graph showing an example of a relation between an inverse number of a transmission length of a scatterer and peripheral brightness.

Fig. 6 is a graph showing an example of a relation between the transmission length of the scatterer and a brightness ratio obtained by dividing the surrounding brightness by the marker brightness.

Fig. 7 is a graph showing an example of a relation between the peripheral brightness and the marker brightness.

Fig. 8 is a view showing an example of the marker brightness and the peripheral brightness when radiation is scattered from perpendicular radiation sources.

Fig. 9 is a graph showing an example of a relation between an estimated scattered radiation coefficient value set by a coefficient value setting unit of the embodiment and a calculated marker brightness.

Fig. 10 is a view showing an example of a template used by a template application unit according to the embodiment.

Fig. 11A is a view showing an example of ranges having candidates for a marker area set by a candidate narrowing unit according to the embodiment.

Fig. 11B is a view showing an example of the ranges having the candidates for the marker area set by the candidate narrowing unit according to the embodiment.

Fig. 12 is a flowchart showing a processing sequence of detecting the marker area in the radiograph by the radiograph analysis device in the embodiment.

## EMBODIMENTS FOR CARRYING OUT THE INVENTION

Hereinafter, while an embodiment of the present invention will be described, the following embodiment is not limited to the present invention applied to the accompanying claims. In addition, all of the combinations of features described in the embodiment are not necessary for the solution to the problem of the present invention.

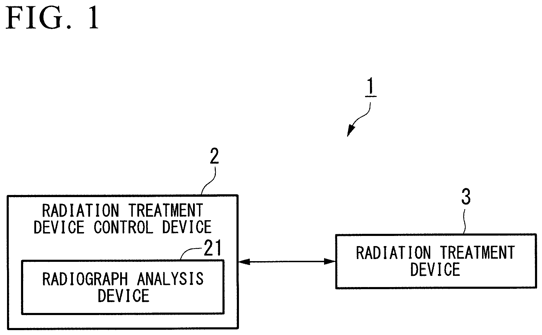

Fig. 1 is a schematic block diagram showing a functional configuration of a radiation treatment system of the embodiment of the present invention. In Fig. 1, the radiation treatment system 1 includes a radiation treatment device control device 2 and a radiation treatment device 3. The radiation treatment device control device 2 includes a radiograph analysis device 21.

The radiation treatment system 1 is a system configured to perform radiation treatment, and specifically, perform exposure of treatment radiation (that may be a baryon beam) or imaging of a radiograph (X-ray radioscopy image) for affected area positioning.

The radiation treatment device control device 2 controls the radiation treatment device 3 to perform exposure of radiation or imaging of radiograph. The radiograph analysis device 21 in the radiation treatment device control device 2 analyzes the radiograph imaged by the radiation treatment device 3, and detects an image (a marker area) of the radiograph of a marker embedded in the vicinity of an affected area in order to position the affected area. For example, a bulb having low X-ray permeability is used as the marker, and the radiograph analysis device 21 detects a shadow of the bulb in the X-ray exposure as the marker area.

The radiation treatment device 3 performs the exposure of the treatment radiation and the imaging of the radiograph according to control of the radiation treatment device control device 2.

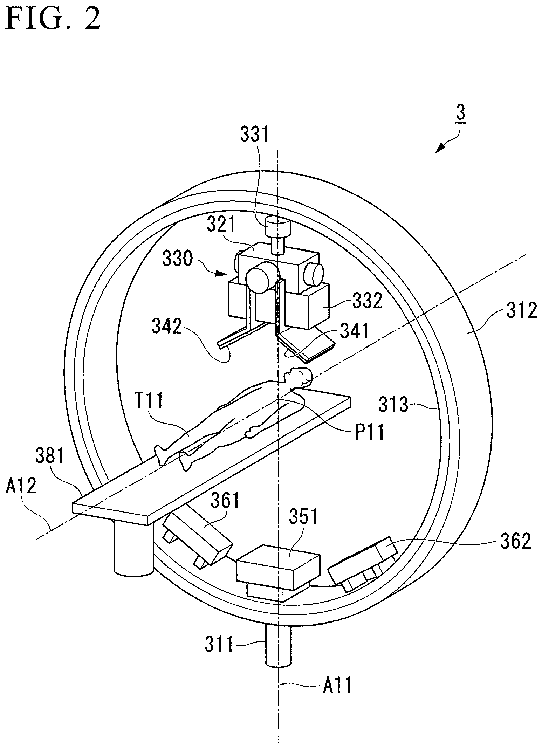

Fig. 2 is a schematic configuration view showing a device configuration of the radiation treatment device 3. In Fig. 2, the radiation treatment device 3 includes a turning driving device 311, an O-ring 312, a traveling gantry 313, an oscillating mechanism 321, an exposure unit 330, sensor arrays 351, 361 and 362 and a couch 381. The exposure unit 330 includes a treatment radiation exposure device 331, a multi-leaf collimator (MLC) 332 and imaging radiation sources 341 and 342.

The turning driving device 311 supports the O-ring 312 to be rotatable about a rotary shaft A11 on a base, and rotates the O-ring 312 according to control of the radiation treatment device control device 2. The rotary shaft A11 is a shaft in a vertical direction.

The O-ring 312 is formed in a ring shape about a rotary shaft A12, and supports the traveling gantry 313 to be rotatable about the rotary shaft A12. The rotary shaft A12 is a shaft in a horizontal direction (that is, a shaft perpendicular to the vertical direction), and is perpendicular to the rotary shaft A11 at an isocenter P11. The rotary shaft A12 is fixed with respect to the O-ring 312. That is, the rotary shaft A12 rotates about the rotary shaft A11 according to rotation of the O-ring 312.

The traveling gantry 313 is formed in a ring shape about the rotary shaft A12 and disposed inside the O-ring 312 to form a concentric circle with respect to the O-ring 312. The radiation treatment device 3 further includes a traveling driving device (not shown), and the traveling gantry 313 rotates about the rotary shaft A12 according to power received from a traveling driving device.

The traveling gantry 313 integrally rotates the respective parts installed at the traveling gantry 313, for example, the imaging radiation source 341 and the sensor array 361, the imaging radiation source 342 and the sensor array 362, or the like, according to rotation thereof.

The oscillating mechanism 321 is fixed inside a ring of the traveling gantry 313, and supports the exposure unit 330 at the traveling gantry 313. The oscillating mechanism 321 varies the direction of the exposure unit 330 according to control of the radiation treatment device control device 2.

The exposure unit 330 is supported by the oscillating mechanism 321 and disposed inside the traveling gantry 313, and emits treatment radiation or imaging radiation.

The treatment radiation exposure device 331 emits the treatment radiation toward an affected area of a patient T11 according to control of the radiation treatment device control device 2.

The multi-leaf collimator 332 matches a shape of an exposure field when the treatment radiation is emitted toward the patient T11 to a shape of the affected area as some of the treatment radiation is blocked according to control of the radiation treatment device control device 2.

The imaging radiation source 341 emits the imaging radiation (X-ray) toward the sensor array 361 according to control of the radiation treatment device control device 2. The imaging radiation source 342 emits the imaging radiation toward the sensor array 362 according to control of the radiation treatment device control device 2. The imaging radiation sources 341 and 342 are fixed to the exposure unit 330 (for example, a housing of the multi-leaf collimator 332) in a direction to which the emitted radiation is perpendicular.

The sensor array 351 is disposed at a position that the treatment radiation from the treatment radiation exposure device 331 reaches, oriented toward the treatment radiation exposure device 331, and fixed inside the ring of the traveling gantry 313. The sensor array 351 receives the treatment radiation passing through the patient T11 or the like as a ray for recognition of the exposure position or recording of the treatment. Further, reception of the ray is reception of the radiation.

The sensor array 361 is disposed at a position that the imaging radiation from the imaging radiation source 341 reaches, oriented toward the imaging radiation source 341, and fixed inside of the ring of the traveling gantry 313. The sensor array 361 receives the imaging radiation emitted from the imaging radiation source 341 and passing through the patient T11 or the like as a ray for the affected area positioning.

The sensor array 362 is disposed at a position that the imaging radiation from the imaging radiation source 342 reaches, oriented toward the imaging radiation source 342, and fixed inside the ring of the traveling gantry 313. The sensor array 362 receives the imaging radiation emitted from the imaging radiation source 342 and passing through the patient T11 or the like as a ray for the affected area positioning.

The couch 381 is used as a member on which the treated patient T11 lies.

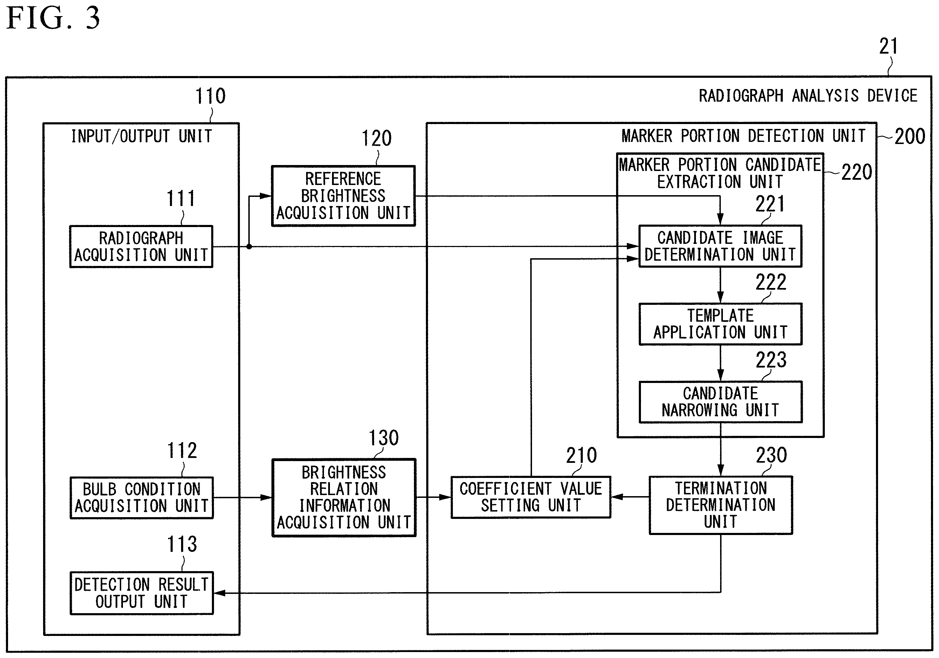

Fig. 3 is a schematic block diagram showing a functional configuration of the radiograph analysis device 21. In Fig. 3, the radiograph analysis device 21 includes an input/output unit 110, a reference brightness acquisition unit 120, a brightness relation information acquisition unit 130 and a marker area detection unit 200. The input/output unit 110 includes a radiograph acquisition unit 111, a bulb condition acquisition unit 112 and a detection result output unit 113. The marker area detection unit 200 includes a coefficient value setting unit 210, a marker area candidate extraction unit 220 and a termination determination unit 230. The marker area candidate extraction unit 220 includes a candidate pixel determination unit 221, a template application unit 222 and a candidate narrowing unit 223.

The input/output unit 110 performs input/output of various data.

The radiograph acquisition unit 111 acquires the radiograph imaged from a specimen in which a marker is embedded. Specifically, the radiograph acquisition unit 111 acquires the radiograph based on the imaging radiation received by the sensor array 361 or the radiograph based on the imaging radiation received by the sensor array 362 as the image data. In particular, the radiograph acquisition unit 111 acquires the radiograph obtained by the imaging radiation sources 341 and 342 simultaneously emitting the radiation and simultaneously imaging the specimen (the vicinity of the affected area of the patient T11) in multiple directions.

The bulb condition acquisition unit 112 acquires information related to a radiation quantity of the radiation from the imaging radiation source 341 or 342. Specifically, the bulb condition acquisition unit 112 acquires a tube voltage and a mAs value (the product of a tube current and an exposure time) as an X-ray bulb condition when the imaging radiation source 341 or 342 emits the imaging radiation.

The detection result output unit 113 outputs a detection result of the radiograph analysis device 21. For example, the detection result output unit 113 outputs coordinate information of the marker detected by the radiograph analysis device 21.

The reference brightness acquisition unit 120 acquires the brightness of the reference portion. The reference brightness disclosed herein is the brightness of the reference portion assumed to be a portion other than the marker in the radiograph. For example, the reference brightness acquisition unit 120 compares the brightness of pixels spaced a predetermined distance from a determination target pixel in four directions, i.e., up, down, right and left from the determination target pixel, upon determination of the candidates for the pixel of the marker area. Then, the reference brightness acquisition unit 120 assumes a pixel having a largest brightness as the portion other than the marker to set a reference portion, and acquires the brightness of the reference portion as the reference brightness.

The brightness relation information acquisition unit 130 acquires brightness relation information. The brightness relation information disclosed herein is information showing a relation between the brightness of the marker area and the brightness of the reference portion. The brightness relation information is generated based on the information related to the radiation quantity of the imaging radiation (specifically, an X-ray bulb condition acquired by the bulb condition acquisition unit 112).

More specifically, the brightness relation information acquisition unit 130 previously stores a function of outputting a determination threshold of the brightness of the marker area using the tube voltage, the mAs value and the brightness of the reference portion as parameters. Then, the brightness relation information acquisition unit 130 substitutes the tube voltage and the mAs value acquired by the bulb condition acquisition unit 112 for the function to acquire a function showing a determination threshold of the brightness of the marker area based on the brightness of the reference portion as the brightness relation information.

Further, the brightness relation information acquisition unit 130 acquires brightness relation information including a coefficient showing an influence of the radiation mixed from the imaging in the other direction. Specifically, the brightness relation information acquisition unit 130 previously stores the function of outputting the determination threshold of the brightness of the marker area using the coefficient showing the influence of the radiation mixed from the imaging in the other direction as the parameter, in addition to the tube voltage, the mAs value and the brightness of the reference portion. Then, the bulb condition acquisition unit 112 substitutes the acquired tube voltage and mAs value for the function to acquire the function showing the determination threshold of the brightness of the marker area as the brightness relation information based on the brightness of the reference portion and the coefficient showing the influence of the radiation mixed from the imaging in the other direction.

Further, hereinafter, the coefficient showing the influence of the radiation mixed from the imaging in the other direction is referred to as "an estimated scattered radiation coefficient" and a value of the coefficient is referred to as "an estimated scattered radiation coefficient value."

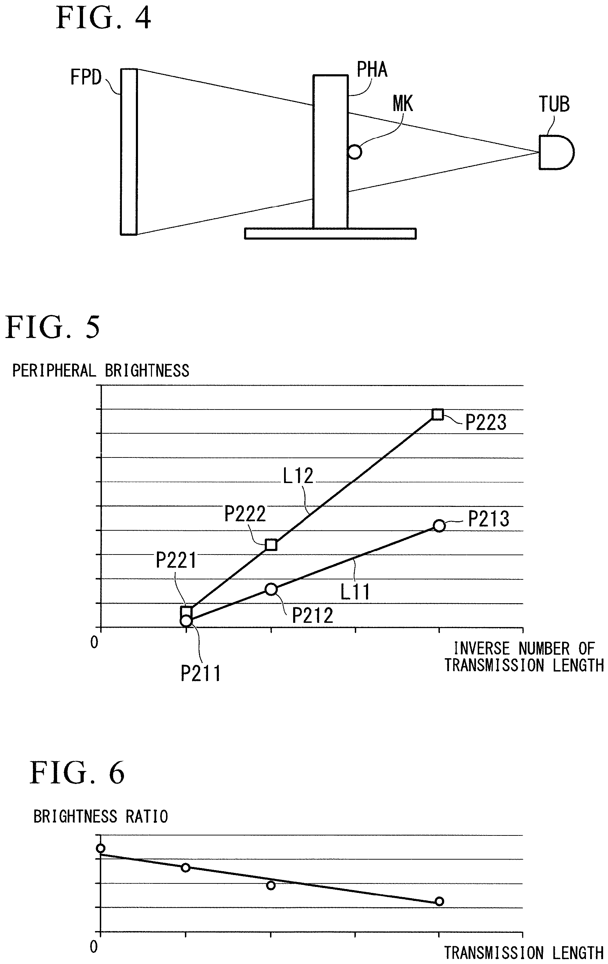

Here, the brightness relation information acquired by the brightness relation information acquisition unit 130 will be described with reference to Figs. 4 to 9.

When the marker area from the radiograph is detected, since the detection using an absolute value of the brightness as a threshold is performed, erroneous detection due to impossibility of dealing with a variation in transmission length of the radiation passing through the human body or the like may occur. That is, when attenuation of the radiation having a short transmission length is relatively small, the brightness is increased throughout the entire radiograph, and the marker area may not be detected as the marker area. On the other hand, when attenuation of the radiation having a large transmission length is relatively large, the brightness is reduced throughout the entire radiograph, and the portion other than the marker may be detected as the marker area.

Here, the brightness of the portion other than the marker is considered to be detected from the radiograph and determination of presence or absence of the marker area is considered to be performed based on a ratio between the determination target area and the portion other than the marker. For example, the portion having the largest brightness in the periphery of the determination target area is considered to be assumed to be a portion other than the marker to use the portion as the reference portion and the target area is considered to be determined as the marker area when Equation (1) is satisfied.

[Math. 1]

$$\frac{I_r}{I_o} \geq A_{conv} \qquad \cdots (1)$$

However, $I_o$ represents the brightness of the determination target area, and $I_r$ represents the brightness of the reference portion. In addition, $A_{conv}$ is the determination threshold, and for example, set to a constant of about 1.3.

When both of the radiation emitted toward the marker area and the radiation emitted toward the portion other than the marker are attenuated at the same ratio when passing through the human body, even though the brightness in the marker area or the portion other than the marker is varied according to the transmission length, the ratio of the brightness is expected to be constant. As a result, the marker area is expected to be able to be precisely detected using Equation (1).

However, in reality, in either the marker area or the portion other than the marker, the brightness is increased by the radiation scattered by the human body. The brightness ratio between the marker area and the portion other than the marker is varied by the transmission length around the marker without a relation in which the radiation emitted toward the marker area and the radiation emitted toward the portion other than the marker are attenuated by the influence of the radiation scattered in the human body at the same ratio when passes the human body.

Here, the following experiment was performed for the purpose of establishing a determination method with higher precision.

Fig. 4 is a view for schematically showing the environment of the experiment. In Fig. 4, a scatterer PHA corresponding to the human body and a marker MK attached to the scatterer PHA are disposed between a radiation source TUB and a sensor array FPD.

In the experimental environment, the transmission length of the scatterer PHA or the X-ray bulb condition was varied to measure the brightness of the marker area (hereinafter referred to as "marker brightness") or the brightness of the portion other than the marker (hereinafter referred to as "peripheral brightness").

Fig. 5 is a graph showing an example of a relation between an inverse number of the transmission length of the scatterer PHA and the peripheral brightness. In Fig. 5, points P211 to P213 represent the relation between the inverse number of the transmission length and the peripheral brightness based on the measurement value when the tube voltage is relatively small, and a line L11 represents an example approximating the straight line of the points P211 to P213. In addition, points P221 to P223 represent the relation between the inverse number of the transmission length and the peripheral brightness based on the measurement value when the tube voltage is relatively large, and a line L12 represents an example approximating the straight line of the points P221 to P223.

Both of the lines L11 or L12 represent that the relation between the inverse number of the transmission length and the peripheral brightness can approximate the straight line. In this way, it was found that the relation between the inverse number of the transmission length and the peripheral brightness can approximate the straight line. Accordingly, a relation between a transmission length t and a peripheral brightness $I_s$ can be approximated by Equation (2).

[Math. 2]

$$I_s = c \cdot \frac{1}{t} + d \quad \cdots (2)$$

Here, a coefficient c is calculated by, for example, Equation (3) based on the X-ray bulb condition.

[Math. 3]

$$c = c_1 \times \left( \frac{V}{V_0} \right)^{c_2} \times \left( \frac{D}{D_0} \right) \quad \cdots (3)$$

Here, V represents the tube voltage of the X-ray bulb, and D represents the mAs value of the X-ray bulb. In addition, Vo represents a constant showing a reference value of the tube voltage of the X-ray bulb, and $D_0$ represents a constant showing a reference value of the mAs value of the X-ray bulb. In addition, $c_1$ and $c_2$ are predetermined constants.

In addition, the coefficient d in Equation (2) is calculated by, for example, Equation (4) based on the X-ray bulb condition.

[Math. 4]

$$d = d_1 \times \left( \frac{V}{V_0} \right)^{d_2} \times \left( \frac{D}{D_0} \right) \quad \cdots (4)$$

However, either $d_1$ or $d_2$ represents a constant. A value of $d_1$ or $d_2$ is obtained through, for example, the experiment.

Equation (2) can be varied like Equation (5).

[Math. 5]

$$t = \frac{c}{I_s - d} \quad \cdots (5)$$

Meanwhile, Fig. 6 is a graph showing an example of the relation between the transmission length of the scatterer PHA and the brightness ratio obtained by dividing the surrounding brightness by the marker brightness.

Fig. 6 shows that the relation between the transmission length and the brightness ratio can approximate the straight line. In this way, it was found that the relation between the transmission length and the brightness ratio can approximate the straight line. Accordingly, a relation between the transmission length t, the peripheral brightness $I_s$ and the marker brightness $I_m$ can approximate Equation (6).

[Math. 6]

$$\frac{I_s}{I_m} = at + b \qquad \cdots (6)$$

Here, a and b represent constants. The values of a and b are obtained through, for example, the experiment.

Equation (5) is substituted into Equation (6) to obtain Equation (7).

[Math. 7]

$$\frac{I_s}{I_m} = a \cdot \frac{c}{I_s - d} + b \qquad \cdots (7)$$

Equation (7) does not include the transmission length t. Like Equation (5) and Equation (6), as a plurality of linear expressions of the transmission length t are acquired, a term of the transmission length can be eliminated. As the equation including the transmission length is used, the radiograph analysis device 21 does not need the information of the transmission length when the process of detecting the marker area is performed. Accordingly, there is no need for a user of the radiograph analysis device 21 to measure the transmission length (the thickness of the specimen).

Equation (7) is solved for $I_m$ to obtain Equation (8).

[Math. 8]

$$I_m = \frac{I_s(I_s - d)}{ac + b(I_s - d)} \qquad \cdots (8)$$

In the case of the imaging in only one direction, a determination threshold for marker area detection based on Equation (8) is considered to be set. For example, as shown in Equation (9), a value obtained by adding a constant $I_{const}$ to a marker brightness $I_m$ of Equation (8) is considered as a determination threshold $I_{thr}$.

[Math. 9]

$$I_{thr} = \frac{I_s(I_s - d)}{ac + b(I_s - d)} + I_{const} \qquad \cdots (9)$$

For example, when only the imaging radiation source 341 and the sensor array 361 perform the imaging while the imaging radiation source 342 and the sensor array 362 do not perform the imaging, the bulb condition acquisition unit 112 acquires the tube voltage and the mAs value of the imaging radiation source 341. Then, the brightness relation information acquisition unit 130 substitutes the tube voltage and the mAs value into Equation (3) and Equation (4) to calculate the values of the coefficients c and d, and substitutes the obtained coefficient value into Equation (9). Equation (9) after substitution of the coefficient values becomes a function of outputting the determination threshold $I_{thr}$ using the surrounding brightness $I_s$ as the parameter.

Here, the candidate pixel determination unit 221 substitutes the reference brightness acquired by the reference brightness acquisition unit 120 for the surrounding brightness $I_s$ of Equation (9) after substitution of the coefficient value, and calculates the determination threshold $I_{thr}$ for the marker area detection.

Further, examples of an approximation equation include Equation (2) and Equation (6), but are not limited thereto.

Another example of the approximation equation of the relation between the transmission length t and the peripheral brightness $I_s$ is shown in Equation (10).

[Math. 10]

$$I_s = c \cdot \exp(-\mu t) + d \quad \cdots (10)$$

In addition, another example of the approximation equation of the relation between the transmission length t, the peripheral brightness $I_s$ and the marker brightness $I_m$ is shown in Equation (11).

[Math. 11]

$$\frac{I_s}{I_m} = a \cdot \exp(bt) \quad \cdots (11)$$

For example, Equation (5) is substituted into Equation (11) to obtain Equation (12).

[Math. 12]

$$\frac{I_s}{I_m} = a \cdot \exp\left( b \cdot \frac{c}{I_s - d} \right) \quad \cdots (12)$$

Equation (12) is solved for $I_m$ to obtain Equation (13).

[Math. 13]

$$I_m = \frac{I_s}{a} \exp\left( -\frac{bc}{I_s - d} \right) \quad \cdots (13)$$

Like the case of Equation (8), for example, a value obtained by adding the constant to the marker brightness $I_m$ in Equation (13) is considered to be the determination threshold $I_{thr}$.

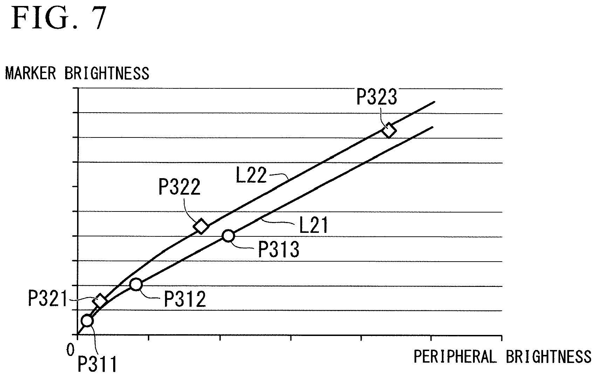

Here, Fig. 7 is a graph showing an example of the relation between the peripheral brightness and the marker brightness. In Fig. 7, points P311 to P313 represent the relation between the peripheral brightness and the marker brightness based on the measurement value when the tube voltage is relatively small, and a line L21 represents a calculated value of Equation (13) in the tube voltage. In addition, points P321 to P323 represent a relation between the peripheral brightness and the marker brightness based on the measurement value when the tube voltage is relatively large, and a line L22 represents a calculated value of Equation (13) in the tube voltage.

The points P311 to P313 and the line L21 substantially coincide with each other. In addition, the points P321 to P323 and the line L22 substantially coincide with each other. In this way, the marker brightness can be precisely calculated using Equation (13). That is, the marker brightness can be precisely estimated based on the brightness of the reference portion. Here, the as the determination threshold for the marker area detection is set to, for example, a value obtained by adding the constant to the estimated value of the marker brightness or an intermediate value of the estimated value of the marker brightness and a lower limit value of the brightness of the portion other than the marker (for example, an average value to a weighted average value), the process of detecting the marker area can be precisely performed.

Next, the determination threshold when the radiation from the perpendicular radiation sources is scattered will be described.

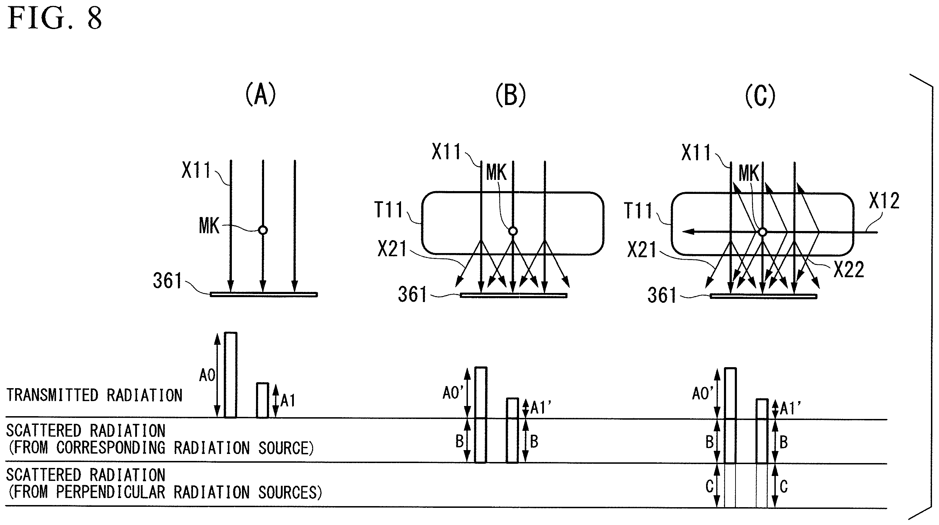

Fig. 8 is a view showing an example of the marker brightness and the peripheral brightness when the radiation from the perpendicular radiation sources is scattered. Further, while Fig. 8 shows the example of the case of the sensor array 361, Fig. 8 is also similar to the sensor array 362.

Fig. 8(A) shows an example when imaging in one direction is performed in a state in which only the marker MK is present while the patient T11 is not present (in the example of Fig. 8, when only the imaging radiation source 341 emits radiation while the imaging radiation source 342 does not emit radiation). In addition, Fig. 8(B) shows an example when the imaging in the one direction is performed in a state in which the patient T11 and the marker MK are present. In addition, Fig. 8(C) shows an example when simultaneous imaging in two directions is performed in a state in which the patient T11 and the marker MK are present (more specifically, when the imaging radiation sources 341 and 342 simultaneously emit the radiation).

In the example of Fig. 8(A), the portion other than the marker reaches a brightness A0 with radiation X11 from the imaging radiation source 341. Meanwhile, in the marker area, the radiation X11 is attenuated by the marker MK, and the brightness reaches a brightness A1. Accordingly, the brightness ratio obtained by dividing the peripheral brightness by the marker brightness becomes A0/A1.

Meanwhile, in the example of Fig. 8(B), the radiation X11 from the imaging radiation source 341 is attenuated by the human body of the patient T11. Meanwhile, radiation X21 scattered in the body of the patient T11 from the radiation X11 also arrives at the sensor array 361. Accordingly, the portion other than the marker reaches the brightness (A0'+B). In addition, the marker area reaches the brightness (A1'+B). Accordingly, the brightness ratio obtained by dividing the peripheral brightness by the marker brightness becomes (A0'+B)/(A1'+B).

In addition, in the example of Fig. 8(C), in addition to the radiation of the case of Fig. 8(B), radiation X22 scattered in the body of the patient T11 from the radiation X12 from the imaging radiation source 342 also arrives at the sensor array 361. Accordingly, the portion other than the marker reaches the brightness (A0'+B+C). In addition, the marker area reaches the brightness (A1'+B+C). Accordingly, the brightness ratio obtained by dividing the peripheral brightness by the marker brightness becomes (A0'+B+C)/(A1'+B+C). In particular, an amount of the radiation X22 scattered in the body of the patient T11 from the radiation X12 from the imaging radiation source 342 is substantially equal to that of the marker area and the portion other than the marker, and thus the brightness ratio obtained by dividing the peripheral brightness by the marker brightness is smaller than that of the case of Fig. 8(B).

Here, like Equation (14), the marker brightness obtained by adding a coefficient (an estimated scattered radiation coefficient) e showing an influence of the radiation mixed from the imaging in the other direction (in the example of Fig. 8, the radiation scattered in the body of the patient T11 from the radiation X12 from the imaging radiation source 342) to the marker brightness $I_m$ of the case of the imaging in only the one direction becomes $I'_m$.

[Math. 14]

$$I'_m = I_m + e \qquad \cdots (14)$$

$I_m$ represents the brightness based on the radiation from the imaging radiation source 341 corresponding to the sensor array 361, e represents the brightness based on the radiation from the perpendicular imaging radiation sources 342, and $I'_m$ represents the brightness obtained by adding $I_m$ and e.

Equation (14) is solved for $I'_m$ to obtain Equation (15).

[Math. 15]

$$I_m = I'_m - e \qquad \cdots (15)$$

In addition, like Equation (16), the peripheral brightness obtained by adding the estimated scattered radiation coefficient e to the peripheral brightness $I_s$ of the case of the imaging in only the one direction is $I'_s$.

[Math. 16]

$$I'_s = I_s + e \qquad \cdots (16)$$

$I_s$ represents the brightness based on the radiation from the imaging radiation source 341 corresponding to the sensor array 361, e represents the brightness based on the radiation from the perpendicular imaging radiation sources 342, and $I'_s$ represents the brightness obtained by adding $I_s$ and e.

Equation (16) is solved for $I'_s$ to obtain Equation (17).

[Math. 17]

$$I_s = I'_s - e \qquad \cdots (17)$$

For example, Equation (15) and Equation (17) are substituted into Equation (8) to obtain Equation (18).

[Math. 18]

$$I'_m = \frac{(I'_s - e)(I'_s - e - d)}{ac + b(I'_s - e - d)} + e \qquad \cdots (18)$$

The right side of Equation (18) is considered to be used as the determination threshold for the marker area detection. In this case, the determination threshold $I_{thr}$ is similar to Equation (19).
[Math. 19]

$$I_{thr} = \frac{(I'_s - e)(I'_s - e - d)}{ac + b(I'_s - e - d)} + e \qquad \cdots (19)$$

For example, when the imaging radiation source 341 and the sensor array 361, and the imaging radiation source 342 and the sensor array 362 simultaneously perform the imaging, with regard to the radiograph imaged by the imaging radiation source 341 and the sensor array 361, the bulb condition acquisition unit 112 acquires the tube voltage and the mAs value of the imaging radiation source 341. Then, the brightness relation information acquisition unit 130 substitutes the tube voltage and the mAs value into Equation (3) and Equation (4) to calculate the values of the coefficients c and d, and substitutes the obtained coefficient values into Equation (19). Equation (19) after substitution of the coefficient values has a function of outputting the determination threshold $I_{thr}$ using the surrounding brightness $I'_s$ and estimated scattered radiation coefficient e as parameters.

Here, the candidate pixel determination unit 221 substitutes the reference brightness acquired by the reference brightness acquisition unit 120 for the surrounding brightness $I'_s$ of Equation (19) after substitution of the coefficient values, and acquires the determination threshold $I_{thr}$ for the marker area detection using the function using the estimated scattered radiation coefficient e as the parameter.

In Equation (19), the calculated value of the marker brightness is used as the determination threshold $I_{thr}$. For this reason, in the radiograph, when the brightness of the marker area is increased by the influence of the scattered light or the like, the candidate pixel determination unit 221 may not extract the marker area. Even in this case, as will be described below, as the coefficient value setting unit 210 increases the value of the estimated scattered radiation coefficient e, the candidate pixel determination unit 221 can extract the marker area.

Further, examples of the determination threshold when the radiation from the perpendicular radiation sources is scattered include Equation (19), but are not limited thereto. For example, Equation (15) and Equation (17) are substituted into Equation (13) to obtain Equation (20).
[Math. 20]

$$I'_m = \frac{I'_s - e}{a}\exp\left(-\frac{bc}{I'_s - e - d}\right) + e \qquad \cdots (20)$$

Like the case of Equation (18), for example, the right side of Equation (20) may be used as the determination threshold $I_{thr}$.

The marker area detection unit 200 detects the marker area based on the brightness relation information acquired by the brightness relation information acquisition unit 130 and the brightness of the reference portion acquired by the reference brightness acquisition unit 120. Specifically, the marker area detection unit 200 detects the portion of the brightness equal to or less than the determination threshold as the marker area based on the determination threshold obtained by substituting the brightness of the reference portion acquired by the reference brightness acquisition unit 120 into the function acquired by the brightness relation information acquisition unit 130.

The coefficient value setting unit 210 sets the value of the estimated scattered radiation coefficient in the brightness relation information acquired by the brightness relation information acquisition unit 130. Then, the coefficient value setting unit 210 varies the value of the coefficient when it is determined that the termination determination unit 230 will not terminate the process of detecting the marker area.

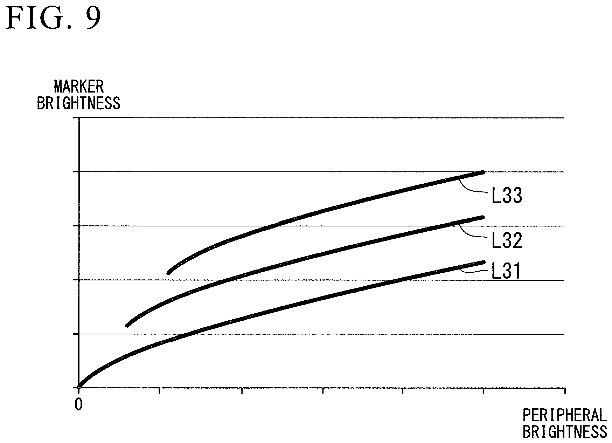

Fig. 9 is a graph showing an example of a relation between the scattered radiation coefficient value set by the coefficient value setting unit 210 and the marker brightness calculated by Equation (20). In Fig. 9, a line L31 represents the marker brightness calculated by Equation (20) when the coefficient value setting unit 210 sets the estimated scattered radiation coefficient value to 0. In addition, a line L32 represents the marker brightness calculated by Equation (20) when the coefficient value setting unit 210 updates the estimated scattered radiation coefficient value from 0 by adding a predetermined value to the estimated scattered radiation coefficient value. The line L32 represents the marker brightness calculated by Equation (20) when the coefficient value setting unit 210 further adds a predetermined value to the estimated scattered radiation coefficient value.

As shown in Fig. 9, the coefficient value setting unit 210 sets the estimated scattered radiation coefficient value to a large value, the calculated marker brightness is increased, and the determination threshold set by the candidate pixel determination unit 221 is also increased. As the determination threshold is increased, the marker area is easily determined, and thus the number of candidates for the marker area extracted by the marker area candidate extraction unit 220 is increased.

Here, the coefficient value setting unit 210 first sets the estimated scattered radiation coefficient value to 0 and gradually increases the estimated scattered radiation coefficient value until the marker area candidate extraction unit 220 extracts the same number or more of candidates for the marker areas as the number of markers.

The marker area candidate extraction unit 220 extracts the candidates for the marker areas based on the brightness relation information having the estimated scattered radiation coefficient value set by the coefficient value setting unit 210 and the brightness of the reference portion acquired by the reference brightness acquisition unit 120. In addition, when the coefficient value setting unit 210 varies the estimated scattered radiation coefficient value, the marker area candidate extraction unit 220 repeats extractions of the candidates for the marker areas based on the estimated scattered radiation coefficient value after the variation.

The candidate pixel determination unit 221 determines whether each pixel of the radiograph is a candidate for a pixel of the marker area based on the brightness relation information acquired by the brightness relation information acquisition unit 130 and the brightness of the reference portion acquired by the reference brightness acquisition unit.

The template application unit 222 extracts the candidates for the marker areas by applying a template including a region of the marker and a region other than the marker to a determination result of the candidate pixel determination unit 221.

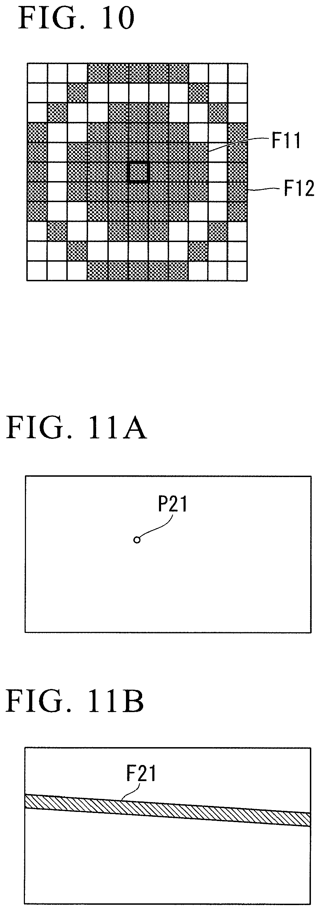

Fig. 10 is a view showing an example of the template used by the template application unit 222.

The template shown in Fig. 10 includes a region F11 of the marker set according to the shape and the size of the marker, and a region F12 other than the marker set to a periphery of the region F11 of the marker.

The template application unit 222 first calculates the average A11 of the brightness of the pixels included in the region F11, and the average A12 of the brightness of the pixels included in the region F12. Next, the template application unit 222 calculates the average A of the calculated averages A11 and A12, and calculates the number $N_d$ of pixels in which the brightness of the pixels included in the region F11 is A or less. When $N_d$ is a threshold or more, that is, when a darker region than the region F12 is present as a circular shape, the template application unit 222 determines a pixel of a center of the region F11 (a pixel shown by a thick line in Fig. 10) as a central position of the candidates for the marker areas.

The candidate narrowing unit 223 sets a range in which the candidates for the marker areas are present in the second image serving as the radiograph imaged in the other direction based on the positions of the candidates for the marker areas in the first image serving as one of the radiographs simultaneously imaged in the multiple directions. Then, when there is no candidate for the marker area in the set range, the candidate narrowing unit 223 eliminates the candidate for the marker area in the first image from the candidates.

Figs. 11A and 11B are views showing an example of a range set by the candidate narrowing unit 223 in which the candidates for the marker areas are present. Fig. 11A shows an example of the candidate for the marker area in the first image (for example, the radiograph obtained by the sensor array 361), and the point P21 shows the candidate for the marker area. In addition, Fig. 11B shows an example of a range set by the candidate narrowing unit 223 in which the candidate for the marker area is present in the second image (for example, the radiograph obtained by the sensor array 362), and a region F21 shows a range in which the candidate for the marker area is present.

When an image of the marker is provided in the first image, while the position of the marker can be identified in a longitudinal direction and a horizontal direction in the first image, the position cannot be identified from only the first image in the depth direction. Accordingly, the position of the marker that can be identified from the first image is a region that connects the radiation source and the sensor array in a 2-dimensional space, for example, a cylindrical region. When the region is projected to the second image, the region becomes, for example, a strip-shaped region like the region F21 of Fig. 11B.

The candidate narrowing unit 223 calculates a range in which the candidate for the marker area is present in the second image (for example, the radiograph obtained by the sensor array 362) based on the positions of the imaging radiation sources 341 and 342, the positions of the sensor arrays 361 and 362, and the position of the candidate for the marker area in the first image (for example, the radiograph obtained by the sensor array 361).

Then, the candidate narrowing unit 223 determines whether the candidate for the marker area is present in the range calculated in the second image.

When there is no candidate for the marker area in the range of the second image, probability that the candidate for the marker area in the first image is not the image of the marker is increased. Here, the candidate narrowing unit 223 eliminates the candidate for the marker area in the first image from the candidates.

Meanwhile, when the candidate for the marker area is present in the range of the second image, probability that the candidate for the marker area in the first image is an image of a real marker is increased. Here, the candidate narrowing unit 223 leaves the candidate for the marker area in the first image as the candidate. That is, no separate processing with respect to the candidate for the marker area is performed.

The termination determination unit 230 compares the preset number of markers with the number of candidates for the marker areas extracted by the marker area candidate extraction unit 220, and determines (decides) whether to terminate the process of detecting the marker area.

Specifically, the termination determination unit 230 receives a user's input about the number of markers previously embedded in the vicinity of the affected area (the number of markers photographed in the radiograph) and previously stores the number of markers. Then, the termination determination unit 230 compares the number of candidates for the marker areas with the previously stored number of markers when the marker area candidate extraction unit 220 extracts the candidates for the marker areas.

When the number of candidates for the marker areas is equal to or larger than the number of markers, it is determined that the termination determination unit 230 terminates the process of detecting the marker areas.

Meanwhile, when the number of candidates for the marker areas is smaller than the number of markers, the termination determination unit 230 determines that the process of detecting the marker areas is not terminated. In this case, as described above, the coefficient value setting unit 210 sets the value of the estimated scattered radiation coefficient to a large value, and the marker area candidate extraction unit 220 performs extraction of the candidates for the marker areas again.

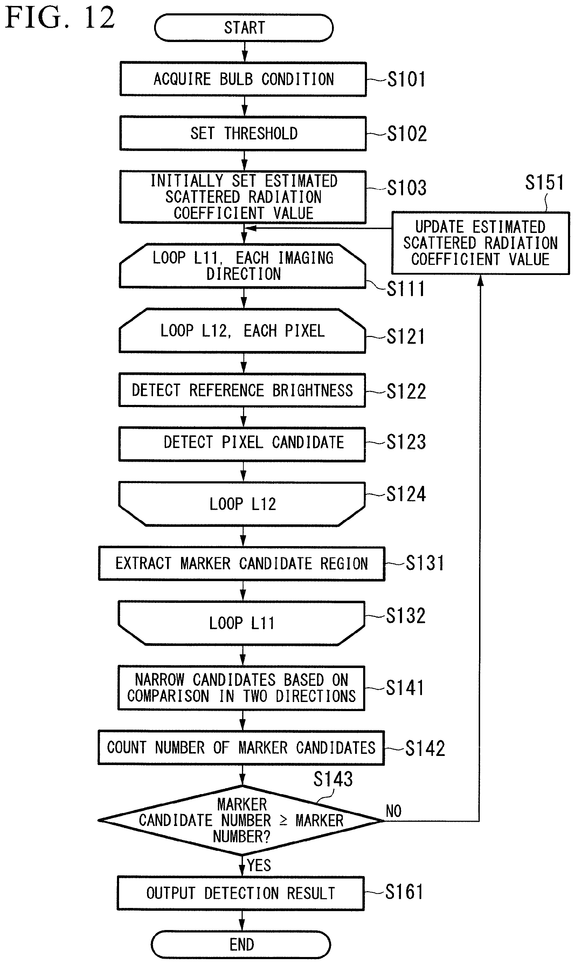

Next, an operation of the radiograph analysis device 21 will be described with reference to Fig. 12. Fig. 12 is a flowchart showing a processing sequence of detecting the marker areas in the radiograph by the radiograph analysis device 21. The radiograph analysis device 21 starts the processing of Fig. 12, for example, when radiograph data obtained by the sensor array 361 and radiograph data obtained by the sensor array 362 are acquired.

In the processing of Fig. 12, first, the bulb condition acquisition unit 112 acquires an X-ray bulb condition (step S101). Specifically, the bulb condition acquisition unit 112 acquires tube voltages and mAs values of the imaging radiation sources 341 and 342 when the radiograph is imaged.

Next, the brightness relation information acquisition unit 130 sets a determination threshold for marker area detection as brightness relation information (step S102). Specifically, the brightness relation information acquisition unit 130 acquires the determination threshold for the marker area detection by a function using a reference brightness and an estimated scattered radiation coefficient value set by the coefficient value setting unit 210 as parameters based on the X-ray bulb condition obtained in step S101.

Then, the coefficient value setting unit 210 initially sets the estimated scattered radiation coefficient value to 0, and substitutes the set estimated scattered radiation coefficient value into a determination threshold set by the brightness relation information acquisition unit 130 (step S103).

Next, the marker area candidate extraction unit 220 starts a loop L11 that performs processing in imaging directions (step S111). That is, in the loop L11, processing with respect to the radiograph based on the imaging radiation received by the sensor array 361 and the radiograph based on the imaging radiation received by the sensor array 362 is performed.

Further, the marker area candidate extraction unit 220 starts a loop L12 that performs processing with respect to pixels included in the radiograph (step S121).

Then, the reference brightness acquisition unit 120 acquires the reference brightness with respect to the pixels that are a processing target in the loop L12 (step S122). Specifically, the reference brightness acquisition unit 120 compares the brightnesses of the pixels spaced a predetermined distance from a determination target pixel in four directions, i.e., up, down, right and left from the determination target pixel, upon determination of the candidates for the pixels of the marker areas, and acquires the largest brightness as the reference brightness.

Next, the candidate pixel determination unit 221 determines whether the pixels that are the processing target in the loop L12 are the candidates for the pixel of the marker area (step S123).

Specifically, the candidate pixel determination unit 221 substitutes the reference brightness detected by the reference brightness acquisition unit 120 in step S122 into the function of the determination threshold set by the brightness relation information acquisition unit 130 in step S102 and for which the coefficient value setting unit 210 substitutes the estimated scattered radiation coefficient value in step S103, and determines (sets) the determination threshold. Then, the candidate pixel determination unit 221 determines whether the brightness of the pixel that is the processing target in the loop L12 is equal to or less than the determination threshold. When it is determined that the brightness is equal to or less than the determination threshold, the candidate pixel determination unit 221 determines that the pixel is the candidate for the pixel of the marker area. Meanwhile, when it is determined that the brightness of the pixel that is the processing target is larger than the determination threshold, the candidate pixel determination unit 221 determines that the pixel is not a candidate for the pixel of the marker area.

Then, the marker area candidate extraction unit 220 determines whether the processing of the loop L12 with respect to all the pixels of the radiograph that are the processing target in the loop L11 has been performed (step S124). When it is determined that there is a pixel on which the processing of the loop L12 has still not been performed, the processing of the loop L12 with respect to the unprocessed pixel is continuously performed. Meanwhile, when it is determined that the processing of the loop L12 with respect to all the pixels has been performed, the loop L12 is terminated.

When the loop L12 is terminated, the template application unit 222 extracts the region of the candidate for the marker area (step S131). For example, the template application unit 222 applies the template described with reference to Fig. 10 with respect to the radiograph that is the processing target in the loop L11, and extracts the entire portion appropriate for the template.

Then, the marker area candidate extraction unit 220 determines whether the processing of the loop L11 in all the imaging directions has been performed (step S132). When it is determined that there is an imaging direction that has still not been processed, the processing of the loop L11 in the unprocessed imaging direction is continuously performed.

Meanwhile, when it is determined that the processing of the loop L11 in all the imaging directions has been determined, the loop L11 is terminated.

When the loop L11 is terminated, the candidate narrowing unit 223 performs narrowing of the candidates for the marker areas (step S141). Specifically, as described with reference to Figs. 11A and 11B, in the radiograph obtained by the sensor array 361 and the radiograph obtained by the sensor array 362, the candidate narrowing unit 223 determines whether the candidate for the marker area corresponding to another image is present in the candidate for the marker area in one image. Then, when it is determined that there is no candidate for the corresponding marker area, the candidate narrowing unit 223 eliminates the candidate for the marker area that is the determination target from the candidates.

Next, the termination determination unit 230 counts the number of candidates for the marker areas extracted by the marker area candidate extraction unit 220 (step S142), and determines whether the number of candidates for the marker area is equal to or larger than the previously stored number of markers (step S143). When it is determined that the number of candidates for the marker areas is smaller than the number or markers (step S143: NO), the coefficient value setting unit 210 updates the estimated scattered radiation coefficient value (step S151). Specifically, the coefficient value setting unit 210 adds a predetermined increment value to a current value of the estimated scattered radiation coefficient. After that, the method returns to step Sill.

Meanwhile, in step S143, when it is determined that the number of candidates for the marker areas is equal to or larger than the number of markers (step S143: YES), the detection result output unit 113 outputs a detection result of the marker area detection unit 200 (step S161). For example, the detection result output unit 113 outputs coordinate information of each of the marker areas using the candidates for the marker areas extracted by the marker area candidate extraction unit 220 as the marker portion of the detection result of the marker area detection unit 200.

After step S161, the processing of Fig. 12 is terminated.

As described above, the brightness relation information acquisition unit 130 acquires the brightness relation information generated based on the information related to the radiation quantity of the radiation emitted by the imaging radiation source 341 or 342 and showing the relation between the brightness of the marker area and the brightness of the reference portion assumed to be a portion other than the marker. In addition, the reference brightness acquisition unit 120 acquires the brightness of the reference portion assumed to be a portion other than the marker. Then, the marker area detection unit 200 detects the marker area based on the brightness relation information acquired by the brightness relation information acquisition unit 130 and the brightness of the reference portion acquired by the reference brightness acquisition unit 120.

Accordingly, the radiograph analysis device 21 can perform the process of detecting the marker area using the determination threshold in which the brightness of the portion other than the marker is reflected. Accordingly, the radiograph analysis device 21 can more accurately reflect a difference between the image brightness of the marker candidate portion and the image brightness of the other portion, and can more precisely detect the marker area.

In addition, the brightness relation information acquisition unit 130 acquires the function set based on the tube voltage, the tube current, the exposure time of the radiation source as the brightness relation information and showing the determination threshold of the brightness of the marker area based on the brightness of the reference portion. Then, the marker area detection unit 200 detects a portion of the brightness equal to or smaller than the determination threshold as the marker area based on the determination threshold obtained by substituting the brightness of the reference portion acquired by the reference brightness acquisition unit 120 for the function acquired by the brightness relation information acquisition unit 130.

In this way, as the brightness relation information acquisition unit 130 acquires the brightness relation information based on the tube voltage, the tube current and the exposure time of the radiation source, for example, as represented in Equation (19), the brightness relation information that does not include the transmission length of the radiation in the specimen as the parameter can be acquired. Accordingly, the radiograph analysis device 21 does not require the information of the transmission length when the process of detecting the marker area is performed. Accordingly, a user of the radiograph analysis device 21 has no need to measure the transmission length (the thickness of the specimen).

In addition, the imaging radiation sources 341 and 342 acquires the radiographs obtained by simultaneously emitting the radiation and simultaneously imaging the specimen in the multiple directions, and the brightness relation information acquisition unit 130 acquires the brightness relation information including a coefficient showing an influence of the radiation mixed from the imaging in another direction. Then, the marker area candidate extraction unit 220 extracts the candidates for the marker area while the coefficient value setting unit 210 gradually increases the estimated scattered radiation coefficient value until the marker areas equal to or larger than the number of markers are detected.

Accordingly, even when the influence of the radiation mixed from the imaging in the other direction is received, the radiograph analysis device 21 can perform the process of detecting the marker area using the determination threshold in which the brightness of the portion other than the marker is reflected. Accordingly, the radiograph analysis device 21 can more accurately reflect the difference between the image brightness of the marker candidate portion and the image brightness of the other portion, and can more precisely detect the marker area.

In addition, the candidate narrowing unit 223 sets a range in which the candidate for the marker area in the second image serving as the radiograph imaged in the other direction is present based on the position of the candidate for the marker area in the first image as one of the radiographs simultaneously imaged in the multiple directions. Then, the candidate narrowing unit 223 eliminates the candidate for the marker area in the first image from the candidates when there is no candidate for the marker area in the set range.

In this way, as the candidate narrowing unit 223 performs the narrowing of the candidates for the marker areas based on the relation between the plurality of images, precision of the process of detecting the marker area performed by the marker area detection unit 200 can be further increased.

In addition, the candidate pixel determination unit 221 determines whether each pixel of the radiograph is the candidate for the pixel of the marker area based on the brightness relation information acquired by the brightness relation information acquisition unit 130 and the brightness of the reference portion acquired by the reference brightness acquisition unit 120. Then, the template application unit 222 applies the template including the region of the marker and the region other than the marker to the determination result of the candidate pixel determination unit 221 to extract the candidates for the marker areas. Accordingly, the shape or the size of the marker can be reflected to the template, and precision of the process of detecting the marker area performed by the marker area detection unit 200 can be further increased.

Further, the process of detecting the marker areas using the technology of the embodiment was tested upon real radiation treatment. As a result, the marker area could be detected at a high detection rate of 99.5%. In addition, even in the positional error of the detected marker, the position of the marker could be identified at a high precision of 0.1 millimeters (mm) to 0.2 millimeters.

Further, as described above, while the case in which the embodiment is applied to the radiation treatment system has been described, the application scope of the embodiment is not limited to the radiation treatment system. For example, the radiograph analysis device 21 may be applied to an affected area observing system that does not involve exposure of the treatment radiation.

Further, the narrowing of the candidates for the marker areas performed by the candidate narrowing unit 223 is not necessary in the embodiment. Accordingly, the radiograph analysis device 21 may not include the candidate narrowing unit 223.

In addition, the template used by the template application unit 222 is not limited the template including the marker region and the region other than the marker, which is described with reference to Fig. 10. For example, the template application unit 222 may use the template including the marker region while not including the region other than the marker.

In addition, acquisition of the reference brightness performed by the reference brightness acquisition unit 120 is not limited to the method of comparing the brightness in four directions, i.e., up, down, right and left, from the above-mentioned determination target pixel. For example, the reference brightness acquisition unit 120 previously stores the region in which the image of the marker is not included in the radiograph, and the reference brightness in the region may be detected.

Further, when the imaging radiation sources 341 and 342 simultaneously emits the radiation and simultaneously images the specimen in the multiple directions, while an angle formed between the radiation emitted by the imaging radiation source 341 and the radiation emitted by the imaging radiation source 342 is typically a right angle, the angle is not limited thereto and may be any arbitrary angle. In addition, the number of imaging radiation sources is not limited to two, and the imaging may be simultaneously performed from three directions or more.

Alternatively, even in the imaging from the one direction, the embodiment can be applied. In this case, the marker area detection unit 200 may be configured to use the determination threshold that does not include the estimated scattered radiation coefficient like Equation (9). In this case, the radiograph analysis device 21 may not include the coefficient value setting unit 210 or the termination determination unit 230.

Alternatively, even in the imaging from the one direction, like the case in which the imaging is simultaneously performed in the multiple directions, the marker area detection unit 200 (the marker area candidate extraction unit 220) may be configured to use the threshold including the estimated scattered radiation coefficient like Equation (19). In this case, like when the imaging is simultaneously performed in the multiple directions, the marker area can be detected with high precision as the marker area candidate extraction unit 220 extracts the candidates for the marker areas while the coefficient value setting unit 210 gradually increases the estimated scattered radiation coefficient value until the number of marker areas equal to or larger than the number of markers is detected.

Further, the radiograph analysis device 21 may acquire the information that limits the position of the marker, and may limit the region in which the marker area is detected in the radiograph. For example, the user of the radiograph analysis device 21 previously registers the position of the marker, and the radiograph analysis device 21 may detect the marker area in only a predetermined range from the registered position of the marker (for example, the range in which movement of the marker is assumed according to breathing or the like of a patient).

Further, a program configured to realize some or all functions of the radiograph analysis device 21 is recorded on a computer-readable recording medium, the program recorded on the recording medium is read by a computer system, and the processing of the components may be performed by executing the program.

Further, "the computer system" disclosed herein may include an operating system (OS), hardware such as peripheral devices or the like.

In addition, "the computer system" may include a homepage providing environment (or a display environment) when a WWW system is used.

In addition, "the computer-readable recording medium" refers a portable storage medium such as a flexible disk, a magneto-optical disc, a ROM, a CD-ROM or the like, and a storage disk such as a hard disk or the like installed in the computer system. Further, "the computer-readable recording medium" includes a medium configured to dynamically hold the program for a short time like a communication wire when the program is sent via a network such as the Internet or the like, or a communication line such as a telephone line or the like, and a medium configured to hold the program for a certain time like a volatile memory in a computer system serving as a server or a client in this case. In addition, the program may be a program configured to execute some of the above-mentioned functions, and further, the above-mentioned functions may be executed in combination with a program previously recorded in the computer system.

Hereinabove, while an embodiment of the present invention has been described with reference to the accompanying drawings, specific configurations are not limited to the embodiment but design modifications or the like may be made without departing from the present invention.

INDUSTRIAL APPLICABILITY

The present invention relates to a radiograph analysis device configured to detect a marker area from a radiograph obtained by imaging a specimen in which a marker is embedded, the radiograph analysis device including a brightness relation information acquisition unit configured to acquire brightness relation information generated based on information related to a quantity of radiation and showing a relation between a brightness of the marker area and a brightness of a reference portion assumed to be a portion other than the marker, a reference brightness acquisition unit configured to acquire the brightness of the reference portion, and a marker area detection unit configured to detect the marker area based on the brightness relation information acquired by the brightness relation information acquisition unit and the brightness of the reference portion acquired by the reference brightness acquisition unit.

According to the present invention, a difference between the image brightness of the marker candidate portion and the image brightness of the other portion can be more accurately reflected.

Reference Symbols

1 Radiation treatment system
2 Radiation treatment device control device
21 Radiograph analysis device
110 Input/output unit
111 Radiograph acquisition unit
112 Bulb condition acquisition unit
113 Detection result output unit
120 Reference brightness acquisition unit
130 Brightness relation information acquisition unit
200 Marker area detection unit
210 Coefficient value setting unit

220 Marker area candidate extraction unit
221 Candidate pixel determination unit
222 Template application unit
223 Candidate narrowing unit
230 Termination determination unit
3 Radiation treatment device

**Claims**

1.  A radiograph analysis device (21) configured to detect a marker area from a radiograph obtained by imaging a specimen in which a marker is embedded, the radiograph analysis device comprising:

    a brightness relation information acquisition unit (130) configured to acquire brightness relation information, the brightness relation information generated based on information related to a quantity of radiation, the brightness relation information showing a relation between a brightness of the marker area and a brightness of a reference portion assumed to be a portion other than the marker;
    a reference brightness acquisition unit (120) configured to acquire the brightness of the reference portion; and
    a marker area detection unit (200) configured to detect the marker area based on the brightness relation information acquired by the brightness relation information acquisition unit (130) and the brightness of the reference portion acquired by the reference brightness acquisition unit (120),
    **characterized in that**
    the brightness relation information acquisition unit (130) is configured to acquire, as the brightness relation information, a function based on the brightness of the reference portion, the function being set based on a tube voltage, a tube current and an exposure time of a radiation source, the function showing a determination threshold of the brightness of the marker area, and
    the marker area detection unit (200) is configured to detect a portion of the brightness equal to or smaller than a determination threshold as the marker area based on the determination threshold obtained by substituting the brightness of the reference portion acquired by the reference brightness acquisition unit (120) for the function acquired by the brightness relation information acquisition unit (130).

2.  The radiograph analysis device (21) according to claim 1, wherein the radiograph is one of the radiographs obtained by simultaneously imaging the specimen in multiple directions,
    the brightness relation information acquisition unit (130) is configured to acquire the brightness relation information including a coefficient showing an influence of radiation mixed from imaging in other direction,
    the marker area detection unit (200) comprises:

    a coefficient value setting unit (210) configured to set a value of the coefficient;
    a marker area candidate extraction unit (220) configured to extract candidates for the marker areas based on the brightness relation information having a coefficient value set by the coefficient value setting unit (210) and the brightness of the reference portion acquired by the reference brightness acquisition unit (120); and
    a termination determination unit (230) configured to determine whether to terminate the process of detecting the marker area by comparing the preset number of markers and the number of candidates for the marker areas extracted by the marker area candidate extraction unit (220),
    the coefficient value setting unit (210) is configured to vary a value of the coefficient when the termination determination unit (230) determines not to terminate the process of detecting the marker area, and
    the marker area candidate extraction unit (220) is configured to extract the candidates for the marker areas based on the value of the coefficient varied by the coefficient value setting unit (210).

3.  The radiograph analysis device (21) according to claim 2, wherein the marker area candidate extraction unit (220) is configured to set a range in which a candidate for a marker area in a second image serving as a radiograph imaged in the other direction is assumed to be present based on the position of the candidate for the marker area in a first image serving as one of the radiographs simultaneously imaged in the multiple directions, the marker area candidate extraction unit eliminating the candidate for the marker area in the first image from the candidates when there is no candidate for the marker area in the set range.

4.  The radiograph analysis device (21) according to claim 1, wherein the marker area detection unit (200) comprises:

a candidate pixel determination unit (221) configured to determine whether each pixel of the radiograph is a candidate for a pixel of the marker area based on the brightness relation information acquired by the brightness relation information acquisition unit (130) and the brightness of the reference portion acquired by the reference brightness acquisition unit (120); and

a template application unit (222) configured to apply a template including a region of a marker and a region other than the marker to a determination result of the candidate pixel determination unit (221) to extract the candidate for the marker area.

5. A radiation treatment system (1) comprising the radiograph analysis device (21) according to claim 1.

6. A marker area detection method of a radiograph analysis device (21) configured to detect a marker area from a radiograph obtained by imaging a specimen in which a marker is embedded, the marker area detection method comprising:

a brightness relation information acquisition step of acquiring brightness relation information generated based on information related to a quantity of radiation and showing a relation between a brightness of the marker area and a brightness of a reference portion assumed to be a portion other than the marker;

a reference brightness acquisition step of acquiring the brightness of the reference portion; and

a marker area detection step of detecting the marker area based on the brightness relation information acquired in the brightness relation information acquisition step and the brightness of the reference portion acquired in the reference brightness acquisition step,

**characterized in that**

as the brightness relation information, a function based on the brightness of the reference portion is acquired in the brightness relation information acquisition step, the function being set based on a tube voltage, a tube current and an exposure time of a radiation source, the function showing a determination threshold of the brightness of the marker area, and

a portion of the brightness equal to or smaller than a determination threshold is detected as the marker area in the marker area detection step based on the determination threshold obtained by substituting the brightness of the reference portion acquired in the reference brightness acquisition step for the function acquired in the brightness relation information acquisition step.

7. A program that causes a computer serving as a radiograph analysis device (21) configured to detect a marker area from a radiograph obtained by imaging a specimen in which a marker is embedded, to execute:

a brightness relation information acquisition step of acquiring brightness relation information generated based on information related to a quantity of radiation and showing a relation between a brightness of the marker area and a brightness of a reference portion assumed to be a portion other than the marker;

a reference brightness acquisition step of acquiring the brightness of the reference portion; and

a marker area detection step of detecting the marker area based on the brightness relation information acquired in the brightness relation information acquisition step and the brightness of the reference portion acquired in the reference brightness acquisition step,

**characterized in that**

as the brightness relation information, a function based on the brightness of the reference portion is acquired in the brightness relation information acquisition step, the function being set based on a tube voltage, a tube current and an exposure time of a radiation source, the function showing a determination threshold of the brightness of the marker area, and

a portion of the brightness equal to or smaller than a determination threshold is detected as the marker area in the marker area detection step based on the determination threshold obtained by substituting the brightness of the reference portion acquired in the reference brightness acquisition step for the function acquired in the brightness relation information acquisition step.

**Patentansprüche**

1. Röntgenanalysevorrichtung (21), die dazu eingerichtet ist, einen Markerbereich aus einer Röntgenaufnahme zu detektieren, indem eine Probe, in der ein Marker eingebettet ist, abgebildet wird, wobei die Röntgenanalysevorrichtung umfasst:

eine Helligkeitsrelationsinformation-Erfassungseinheit (130), die dazu ausgelegt ist, Helligkeitsrelationsinformationen zu erfassen, wobei die Helligkeitsrelationsinformationen auf der Grundlage von sich auf eine Strahlungsmenge beziehenden Informationen erzeugt worden sind, wobei die Helligkeitsrelationsinformationen eine Relation zwischen einer Helligkeit des Markerbereichs und einer Helligkeit eines Referenzabschnitts, der annahmegemäß ein anderer Abschnitt als der des Markers ist, zeigen;

eine Referenzhelligkeit-Erfassungseinheit (120), die dazu eingerichtet ist, die Helligkeit des Referenzabschnitts zu erfassen; und

eine Markerbereich-Detektionseinheit (200), die dazu eingerichtet ist, den Markerbereich auf der Grundlage der von der Helligkeitsrelationsinformation-Erfassungseinheit (130) erfassten Helligkeitsrelationsinformationen und der von der Referenzhelligkeit-Erfassungseinheit (120) erfassten Helligkeit des Referenzabschnitts zu detektieren;

**gekennzeichnet dadurch, dass**

die Helligkeitsrelationsinformation-Erfassungseinheit (130) dazu eingerichtet ist, als die Helligkeitsrelationsinformationen eine auf der Helligkeit des Referenzabschnitts basierende Funktion zu erfassen, wobei die Funktion auf der Grundlage einer Röhrenspannung, eines Röhrenstroms und einer Aussetzungszeit einer Strahlungsquelle eingestellt ist, wobei die Funktion einen Bestimmungsschwellenwert der Helligkeit des Markerbereichs zeigt, und

die Markerbereich-Detektionseinheit (200) dazu eingestellt ist, einen Abschnitt der gleichen oder kleineren Helligkeit im Vergleich zu einem Bestimmungsschwellenwert als den Markerbereich auf der Grundlage des Bestimmungsschwellenwerts zu detektieren, der durch Ersetzen der Helligkeit des Referenzabschnitts, die von der Referenzhelligkeit-Erfassungseinheit (120) erfasst worden ist, für die von der Helligkeitsrelationsinformation-Erfassungseinheit (130) erfasste Funktion erhalten worden ist.

2. Röntgenanalysevorrichtung (21) nach Anspruch 1, wobei die Röntgenaufnahme eine von durch gleichzeitiges Abbilden der Probe in mehreren Richtungen erhaltenen Röntgenaufnahmen ist,

wobei die Helligkeitsrelationsinformation-Erfassungseinheit (130) dazu eingerichtet ist, die Helligkeitsrelationsinformationen zu erfassen, die einen Koeffizienten enthalten, der einen Einfluss von Strahlung zeigt, die vom Abbilden in einer anderen Richtung vermischt ist,

wobei die Markerbereich-Detektionseinheit (200) umfasst:

eine Koeffizientenwert-Einstelleinheit (210), die dazu eingerichtet ist, einen Wert des Koeffizienten einzustellen;

eine Markerbereich-Kandidatenextraktionseinheit (220), die dazu eingerichtet ist, Kandidaten für die Markerbereiche auf der Grundlage der Helligkeitsrelationsinformationen mit einem von der Koeffizientenwert-Einstelleinheit (210) eingestellten Koeffizientenwert und der von der Referenzhelligkeit-Erfassungseinheit (120) erfassten Helligkeit des Referenzabschnitts zu extrahieren; und

eine Beendigungsbestimmungseinheit (230), die zu bestimmen eingerichtet ist, ob der Prozess des Detektierens des Markerbereichs zu beenden ist, indem die voreingestellte Anzahl von Markern und die Anzahl von von der Markerbereich-Kandidatenextraktionseinheit (220) extrahierten Kandidaten für die Markerbereiche verglichen werden,

wobei die Koeffizientenwert-Einstelleinheit (210) dazu eingerichtet ist, einen Wert des Koeffizienten zu variieren, wenn die Beendigungsbestimmungseinheit (230) bestimmt, dass der Prozess des Detektierens des Markerbereichs nicht zu beenden ist, und

wobei die Markerbereich-Kandidatenextraktionseinheit (220) dazu eingerichtet ist, die Kandidaten für die Markerbereiche auf der Grundlage des von der Koeffizientenwert-Einstelleinheit (210) variierten Werts des Koeffizienten zu extrahieren.

3. Röntgenanalysevorrichtung (21) nach Anspruch 2, wobei die Markerbereich-Kandidatenextraktionseinheit (220) dazu eingerichtet ist, einen Eingrenzungsbereich einzustellen, in dem ein Kandidat für einen Markerbereich in einem zweiten Bild, das als eine in der anderen Richtung abgebildete Röntgenaufnahme dient, auf der Grundlage der Position des Kandidaten für den Markerbereich in einem ersten Bild, das als eine der gleichzeitig in den mehreren Richtungen abgebildeten Röntgenaufnahmen dient, als vorhanden angenommen wird, wobei die Markerbereich-Kandidatenextraktionseinheit den Kandidaten für den Markerbereich im ersten Bild aus den Kandidaten eliminiert, wenn es im eingestellten Eingrenzungsbereich keinen Kandidaten für den Markerbereich gibt.

4. Röntgenanalysevorrichtung (21) nach Anspruch 1, wobei die Markerbereich-Detektionseinheit (200) umfasst:

eine Kandidatenpixel-Bestimmungseinheit (221), die zu bestimmen eingerichtet ist, ob jedes Pixel der Röntgenaufnahme ein Kandidat für ein Pixel des Markerbereichs auf der Grundlage der von der Helligkeitsrelationsinformation-Erfassungseinheit (130) erfassten Helligkeitsrelationsinformationen und der von der Referenzhelligkeit-Erfassungseinheit (120) erfassten Helligkeit des Referenzabschnitts ist; und
eine Vorlagenanwendungseinheit (222), die dazu eingerichtet ist, eine Vorlage, die eine Region eines Markers und eine andere Region als die des Markers enthält, auf ein Bestimmungsergebnis der Kandidatenpixel-Bestimmungseinheit (221) anzuwenden, um den Kandidaten für den Markerbereich zu extrahieren.

**5.** Röntgenbehandlungssystem (1), das die Röntgenanalysevorrichtung (21) nach Anspruch 1 umfasst.

**6.** Markerbereich-Detektionsverfahren einer Röntgenanalysevorrichtung (21), das dazu eingerichtet ist, einen Markerbereich aus einer Röntgenaufnahme zu detektieren, die durch Abbilden einer Probe, in der ein Marker eingebettet ist, erhalten worden ist, wobei das Markerbereich-Detektionsverfahren umfasst:

einen Helligkeitsrelationsinformation-Erfassungsschritt, bei dem Helligkeitsrelationsinformationen erfasst werden, die auf der Grundlage von sich auf eine Strahlungsmenge beziehenden Informationen erzeugt worden sind und eine Relation zwischen einer Helligkeit des Markerbereichs und einer Helligkeit eines Referenzabschnitts, der annahmegemäß ein anderer Abschnitt als der des Markers ist, zeigen;
einen Referenzhelligkeit-Erfassungsschritt, bei dem die Helligkeit des Referenzabschnitts erfasst wird; und
einen Markerbereich-Detektionsschritt, bei dem der Markerbereich auf der Grundlage der im Helligkeitsrelationsinformation-Erfassungsschritt erfassten Helligkeitsrelationsinformationen und der im Referenzhelligkeit-Erfassungsschritt erfassten Helligkeit des Referenzabschnitts detektiert wird,
**gekennzeichnet dadurch, dass**
als die Helligkeitsrelationsinformationen eine Funktion auf der Grundlage der Helligkeit des Referenzabschnitts im Helligkeitsrelationsinformation-Erfassungsschritt erfasst wird, wobei die Funktion auf der Grundlage einer Röhrenspannung, eines Röhrenstroms und einer Aussetzungszeit einer Strahlungsquelle eingestellt wird, wobei die Funktion einen Bestimmungsschwellenwert der Helligkeit des Markerbereichs zeigt, und
ein Abschnitt der gleichen oder kleineren Helligkeit im Vergleich zu einem Bestimmungsschwellenwert als der Markerbereich im Markerbereich-Detektionsschritt auf der Grundlage des Bestimmungsschwellenwerts detektiert wird, der durch Ersetzen der im Referenzhelligkeit-Erfassungsschritt erfassten Helligkeit des Referenzabschnitts für die im Helligkeitsrelationsinformation-Erfassungsschritt erfasste Funktion erhalten wird.

**7.** Programm, das einen Computer, der als eine Röntgenanalysevorrichtung (21) dient, die dazu eingerichtet ist, einen Markerbereich aus einer Röntgenaufnahme zu detektieren, die durch Abbilden einer Probe, in der ein Marker eingebettet ist, erhalten wird, ausführen lässt:

einen Helligkeitsrelationsinformation-Erfassungsschritt, bei dem Helligkeitsrelationsinformationen erfasst werden, die auf der Grundlage von sich auf eine Strahlungsmenge beziehenden Informationen erzeugt worden sind und eine Relation zwischen einer Helligkeit des Markerbereichs und einer Helligkeit eines Referenzabschnitts, der annahmegemäß ein anderer Abschnitt als der des Markers ist, zeigen;
einen Referenzhelligkeit-Erfassungsschritt, bei dem die Helligkeit des Referenzabschnitts erfasst wird; und
einen Markerbereich-Detektionsschritt, bei dem der Markerbereich auf der Grundlage der im Helligkeitsrelationsinformation-Erfassungsschritt erfassten Helligkeitsrelationsinformationen und der im Referenzhelligkeit-Erfassungsschritt erfassten Helligkeit des Referenzabschnitts detektiert wird,
**gekennzeichnet dadurch, dass**
als die Helligkeitsrelationsinformationen eine Funktion auf der Grundlage der Helligkeit des Referenzabschnitts im Helligkeitsrelationsinformation-Erfassungsschritt erfasst wird, wobei die Funktion auf der Grundlage einer Röhrenspannung, eines Röhrenstroms und einer Aussetzungszeit einer Strahlungsquelle eingestellt wird, wobei die Funktion einen Bestimmungsschwellenwert der Helligkeit des Markerbereichs zeigt, und
ein Abschnitt der gleichen oder kleineren Helligkeit im Vergleich zu einem Bestimmungsschwellenwert als der Markerbereich im Markerbereich-Detektionsschritt auf der Grundlage des Bestimmungsschwellenwerts detektiert wird, der durch Ersetzen der im Referenzhelligkeit-Erfassungsschritt erfassten Helligkeit des Referenzabschnitts für die im Helligkeitsrelationsinformation-Erfassungsschritt erfasste Funktion erhalten wird.

**Revendications**

**1.** Dispositif (21) d'analyse radiographique configuré pour détecter une zone de marqueur à partir d'une radiographie obtenue par imagerie d'un spécimen dans lequel un marqueur est intégré, le dispositif d'analyse radiographique comprenant :

une unité (130) d'acquisition d'information de relation de luminosités configurée pour acquérir une information de relation de luminosités, l'information de relation de luminosités générée sur la base d'une information se rapportant à une quantité de rayonnement, l'information de relation de luminosités montrant une relation entre une luminosité de la zone de marqueur et une luminosité d'une partie de référence supposée être une partie autre que le marqueur ;

une unité (120) d'acquisition de luminosité de référence configurée pour acquérir la luminosité de la partie de référence ; et

une unité (200) de détection de zone de marqueur configurée pour détecter la zone de marqueur sur la base de l'information de relation de luminosités acquise par l'unité (130) d'acquisition d'information de relation de luminosités et de la luminosité de la partie de référence acquise par l'unité (120) d'acquisition de luminosité de référence,

**caractérisé en ce que**

l'unité (130) d'acquisition d'information de relation de luminosités est configurée pour acquérir, comme l'information de relation de luminosités, une fonction basée sur la luminosité de la partie de référence, la fonction étant définie sur la base d'une tension de tube, d'un courant de tube et d'une durée d'exposition d'une source de rayonnement, la fonction montrant un seuil de détermination de la luminosité de la zone de marqueur, et

l'unité (200) de détection de zone de marqueur est configurée pour détecter une partie de la luminosité égale ou inférieure à un seuil de détermination comme la zone de marqueur sur la base du seuil de détermination obtenu en remplaçant la luminosité de la partie de référence acquise par l'unité (120) d'acquisition de luminosité de référence par la fonction acquise par l'unité (130) d'acquisition d'information de relation de luminosités.

2. Dispositif (21) d'analyse radiographique selon la revendication 1, dans lequel la radiographie est une des radiographies obtenues par imagerie simultanée du spécimen dans des directions multiples,

l'unité (130) d'acquisition d'information de relation de luminosités est configurée pour acquérir l'information de relation de luminosités incluant un coefficient montrant une influence de rayonnement mélangé à partir d'une imagerie dans une autre direction,

l'unité (200) de détection de zone de marqueur comprend :

une unité (210) de définition de valeur de coefficient configurée pour définir une valeur du coefficient ;

une unité (220) d'extraction de zones de marqueur candidates configurée pour extraire des candidates pour les zones de marqueur sur la base de l'information de relation de luminosités ayant une valeur de coefficient définie par l'unité (210) de définition de valeur de coefficient et de la luminosité de la partie de référence acquise par l'unité (120) d'acquisition de luminosité de référence ; et

une unité (230) de détermination de fin configurée pour déterminer s'il convient de mettre fin au processus de détection de la zone de marqueur en comparant le nombre prédéfini de marqueurs et le nombre de candidates pour les zones de marqueur extrait par l'unité (220) d'extraction de zones de marqueur candidates,

l'unité (210) de définition de valeur de coefficient est configurée pour faire varier une valeur du coefficient lorsque l'unité (230) de détermination de fin détermine de ne pas mettre fin au processus de détection de la zone de marqueur, et

l'unité (220) d'extraction de zones de marqueur candidates est configurée pour extraire les candidates pour les zones de marqueur sur la base de la valeur du coefficient fait varier par l'unité (210) de définition de valeur de coefficient.

3. Dispositif (21) d'analyse radiographique selon la revendication 2, dans lequel l'unité (220) d'extraction de zones de marqueur candidates est configurée pour définir une plage dans laquelle une candidate pour une zone de marqueur dans une deuxième image servant comme une radiographie imagée dans l'autre direction est supposée être présente sur la base de la position de la candidate pour la zone de marqueur dans une première image servant comme une des radiographies imagées simultanément dans les directions multiples, l'unité d'extraction de zones de marqueur candidates éliminant la candidate pour la zone de marqueur dans la première image des candidates lorsqu'il n'y a pas de candidate pour la zone de marqueur dans la plage définie.

4. Dispositif (21) d'analyse radiographique selon la revendication 1, dans lequel l'unité (200) de détection de zone de marqueur comprend :

une unité (221) de détermination de pixels candidats configurée pour déterminer si chaque pixel de la radiographie est un candidat pour un pixel de la zone de marqueur sur la base de l'information de relation de luminosités acquise par l'unité (130) d'acquisition d'information de relation de luminosités et de la luminosité de la partie de référence acquise par l'unité (120) d'acquisition de luminosité de référence ; et

une unité (222) d'application de modèle configurée pour appliquer un modèle incluant une région d'un marqueur et une région autre que le marqueur à un résultat de détermination de l'unité (221) de détermination de pixels candidats pour extraire le candidat pour la zone de marqueur.

5. Système (1) de radiothérapie comprenant le dispositif (21) d'analyse radiographique selon la revendication 1.

6. Procédé de détection de zone de marqueur d'un dispositif (21) d'analyse radiographique configuré pour détecter une zone de marqueur à partir d'une radiographie obtenue par imagerie d'un spécimen dans lequel un marqueur est intégré, le procédé de détection de zone de marqueur comprenant :

une étape d'acquisition d'information de relation de luminosités pour acquérir une information de relation de luminosités générée sur la base d'une information se rapportant à une quantité de rayonnement et montrant une relation entre une luminosité de la zone de marqueur et une luminosité d'une partie de référence supposée être une partie autre que le marqueur ;
une étape d'acquisition de luminosité de référence pour acquérir la luminosité de la partie de référence ; et
une étape de détection de zone de marqueur pour détecter la zone de marqueur sur la base de l'information de relation de luminosités acquise à l'étape d'acquisition d'information de relation de luminosités et de la luminosité de la partie de référence acquise à l'étape d'acquisition de luminosité de référence,
**caractérisé en ce que**
comme l'information de relation de luminosités, une fonction basée sur la luminosité de la partie de référence est acquise à l'étape d'acquisition d'information de relation de luminosités, la fonction étant définie sur la base d'une tension de tube, d'un courant de tube et d'une durée d'exposition d'une source de rayonnement, la fonction montrant un seuil de détermination de la luminosité de la zone de marqueur, et
une partie de la luminosité égale ou inférieure à un seuil de détermination est détectée comme la zone de marqueur à l'étape de détection de zone de marqueur sur la base du seuil de détermination obtenu en remplaçant la luminosité de la partie de référence acquise à l'étape d'acquisition de luminosité de référence par la fonction acquise à l'étape d'acquisition d'information de relation de luminosités.

7. Programme qui fait en sorte qu'un ordinateur servant comme un dispositif (21) d'analyse radiographique configuré pour détecter une zone de marqueur à partir d'une radiographie obtenue par imagerie d'un spécimen dans lequel un marqueur est intégré, exécute :

une étape d'acquisition d'information de relation de luminosités pour acquérir une information de relation de luminosités générée sur la base d'une information se rapportant à une quantité de rayonnement et montrant une relation entre une luminosité de la zone de marqueur et une luminosité d'une partie de référence supposée être une partie autre que le marqueur ;
une étape d'acquisition de luminosité de référence pour acquérir la luminosité de la partie de référence ; et
une étape de détection de zone de marqueur pour détecter la zone de marqueur sur la base de l'information de relation de luminosités acquise à l'étape d'acquisition d'information de relation de luminosités et de la luminosité de la partie de référence acquise à l'étape d'acquisition de luminosité de référence,
**caractérisé en ce que**
comme l'information de relation de luminosités, une fonction basée sur la luminosité de la partie de référence est acquise à l'étape d'acquisition d'information de relation de luminosités, la fonction étant définie sur la base d'une tension de tube, d'un courant de tube et d'une durée d'exposition d'une source de rayonnement, la fonction montrant un seuil de détermination de la luminosité de la zone de marqueur, et
une partie de la luminosité égale ou inférieure à un seuil de détermination est détectée comme la zone de marqueur à l'étape de détection de zone de marqueur sur la base du seuil de détermination obtenu en remplaçant la luminosité de la partie de référence acquise à l'étape d'acquisition de luminosité de référence par la fonction acquise à l'étape d'acquisition d'information de relation de luminosités.

# FIG. 1

1

2

RADIATION TREATMENT
DEVICE CONTROL DEVICE

21

RADIOGRAPH ANALYSIS
DEVICE

3

RADIATION TREATMENT
DEVICE

FIG. 2

# FIG. 3

RADIOGRAPH ANALYSIS DEVICE — 21

**200** MARKER PORTION DETECTION UNIT

**110** INPUT/OUTPUT UNIT

**120** REFERENCE BRIGHTNESS ACQUISITION UNIT

**111** RADIOGRAPH ACQUISITION UNIT

**220** MARKER PORTION CANDIDATE EXTRACTION UNIT

**221** CANDIDATE IMAGE DETERMINATION UNIT

**222** TEMPLATE APPLICATION UNIT

**223** CANDIDATE NARROWING UNIT

**112** BULB CONDITION ACQUISITION UNIT

**130** BRIGHTNESS RELATION INFORMATION ACQUISITION UNIT

**210** COEFFICIENT VALUE SETTING UNIT

**230** TERMINATION DETERMINATION UNIT

**113** DETECTION RESULT OUTPUT UNIT

# FIG. 4

# FIG. 5

PERIPHERAL BRIGHTNESS

INVERSE NUMBER OF
TRANSMISSION LENGTH

# FIG. 6

BRIGHTNESS RATIO

TRANSMISSION LENGTH

FIG. 7

MARKER BRIGHTNESS

P323

P322

L22

L21

P313

P321

P312

0
P311

PERIPHERAL BRIGHTNESS

EP 3 006 085 B1

FIG. 8

(A)

X11

MK

361

(B)

X11
MK
X21

361

T11

(C)

X12
X22
X21

T11

MK
X11

361

TRANSMITTED RADIATION

A0

A1

B

SCATTERED RADIATION
(FROM CORRESPONDING RADIATION SOURCE)

SCATTERED RADIATION
(FROM PERPENDICULAR RADIATION SOURCES)

A0'

A1'

B

A0'

A1'

B

C

C

FIG. 9

# FIG. 10

# FIG. 11A

# FIG. 11B

FIG. 12

```
                        ┌─────────────┐
                        │    START    │
                        └──────┬──────┘
                               │
              ┌────────────────────────────────┐
              │     ACQUIRE BULB CONDITION      │─S101
              └────────────────┬───────────────┘
                               │
              ┌────────────────────────────────┐
              │         SET THRESHOLD           │─S102
              └────────────────┬───────────────┘
                               │
              ┌────────────────────────────────┐          S151
              │    INITIALLY SET ESTIMATED      │─S103       │
              │     SCATTERED RADIATION         │    ┌──────────────────┐
              │     COEFFICIENT VALUE           │    │ UPDATE ESTIMATED │
              └────────────────┬───────────────┘    │ SCATTERED RADIATION│
                               │      ◄─────────────│ COEFFICIENT VALUE │
             ╱──────────────────────────────────╲   └──────────────────┘
            ╱   LOOP L11,  EACH IMAGING           ╲
            ╲        DIRECTION                     ╱─S111
             ╲──────────────────────────────────╱
                               │
             ╱──────────────────────────────────╲
            ╱    LOOP L12,  EACH PIXEL            ╲─S121
             ╲──────────────────────────────────╱
                               │
              ┌────────────────────────────────┐
              │   DETECT REFERENCE BRIGHTNESS    │─S122
              └────────────────┬───────────────┘
                               │
              ┌────────────────────────────────┐
              │      DETECT PIXEL CANDIDATE      │─S123
              └────────────────┬───────────────┘
                               │
              ╲──────────────────────────────────╱
               ╲          LOOP L12                ╱─S124
                ╲────────────────────────────────╱
                               │
              ┌────────────────────────────────┐
              │   EXTRACT MARKER CANDIDATE REGION│─S131
              └────────────────┬───────────────┘
                               │
              ╲──────────────────────────────────╱
               ╲          LOOP L11                ╱─S132
                ╲────────────────────────────────╱
                               │
              ┌────────────────────────────────┐
              │   NARROW CANDIDATES BASED ON     │─S141
              │  COMPARISON IN TWO DIRECTIONS    │
              └────────────────┬───────────────┘
                               │
              ┌────────────────────────────────┐
              │ COUNT NUMBER OF MARKER CANDIDATES│─S142
              └────────────────┬───────────────┘
                               │        S143
                          ╱────────────╲
                         ╱    MARKER     ╲   NO
              ◄─────────  CANDIDATE NUMBER ≥ MARKER
                         ╲    NUMBER?     ╱
                          ╲────────────╱
                               │ YES
              ┌────────────────────────────────┐
              │      OUTPUT DETECTION RESULT     │─S161
              └────────────────┬───────────────┘
                               │
                        ┌─────────────┐
                        │     END     │
                        └─────────────┘
```

**EP 3 006 085 B1**

**REFERENCES CITED IN THE DESCRIPTION**

**Patent documents cited in the description**

- JP 3053389 A
- US 2005059887 A1
- JP 2012170767 A